# EUROPÄISCHE PATENTANMELDUNG

(11) **EP 1 925 661 A1**
(43) Veröffentlichungstag der Anmeldung: **28.05.2008**
(21) Anmeldenummer: 06024360.7
(22) Anmeldetag: 24.11.2006
(51) Int. Cl.: C12M 1/107, C12M 1/113

(54) **Herstellungsverfahren eines Biomasse-Futters für Bakterien sowie seine Verwendung in Biogasanlagen**

(71) Anmelder: UTS Biogastechnik GmbH, 84419 Obertaufkirchen (DE)
(72) Erfinder: Bürger, Adam, 83527 Haag (DE)
(74) Vertreter: Liebl, Thomas

(57) **Zusammenfassung**

Die Erfindung betrifft ein Herstellverfahren zur Herstellung eines Biomasse-Futters für Bakterien, ein Biomasse-Futter für Bakterien und ein Betriebsverfahren für eine Biogasanlage unter Verwendung des Biomasse-Futters. In einem Fermenter (2) einer einstufigen, im Durchflussverfahren arbeitenden Biogasanlage (1) wird ein Substrat mit einem Trockensubstanzgehalt in einem Nassfermentationsprozess vergoren. Ein solches Substrat wird als Entnahmesubstrat dem Fermenter (2) während des Betriebs entnommen, und zumindest teilweise einem Separator (6) zugeführt, in dem eine zumindest teilweise Trennung in einen Substrat-Feststoffanteil und einen Substrat-Flüssiganteil erfolgt. In einem weiteren Verfahrensschritt wird zumindest einem Teil des separierten Substrat-Feststoffanteils wenigstens ein Futterergänzungsmittel in der Art eines Mittels zur Erhöhung des Nährstoff-/Energiepotentials und/oder eines Mittels zur Appetitanregung für die Bakterien und/oder eines Mittels zur Gesundheitsförderung für die Bakterien zugesetzt, wodurch ein hochwertiges Biomasse-Futter als Bio-Kraftfutter für Bakterien gebildet wird.

## Beschreibung

Die Erfindung betrifft ein Herstellverfahren zur Herstellung eines Biomasse-Futters für Bakterien, ein Biomasse-Futter für Bakterien und ein Betriebsverfahren für eine Biogasanlage unter Verwendung des Biomasse-Futters.

Die Biogasgewinnung zur Energieerzeugung gewinnt vorrangig im landwirtschaftlichen Bereich zunehmend an Bedeutung. Die Gründe dafür sind im wesentlichen die Erschließung neuer Einkommensquellen in der Landwirtschaft durch eine staatlich geförderte regenerative und umweltverträgliche Energiegewinnung. Die dafür bekannten Biogasanlagen sind meist für einen kontinuierlichen einstufigen Betrieb ausgelegt, mit einem Fermenterbehälter, in dem in einem anaeroben Nassvergärungsprozess zur Gewinnung von Biogas Biomassen als anorganische Substrate vergoren werden. Je nach den Gegebenheiten sind einem solchen Fermenter ein Nachfermenter und ein Endlager sowie separate Gasspeicher für das erzeugte Biogas nachgeordnet. Zur Beschickung des Fermenterbehälters mit vergärbaren Stoffen ist diesem eine meist automatisierte Fütterungseinrichtung vorgeschaltet, wobei für flüssige Substratbestandteile Pumpen und für feste Biomassen Feststoff-Zudosiereinrichtungen mit Wägeeinrichtungen und Befüllschnecken oder Förderbändern verwendet werden. Zudem sind in den Behältern und Fermentern Rühreinrichtungen für eine gleichmäßige Verteilung der Substratbestandteile angebracht (EP 1 251 165 A1). Ein wesentlicher Aspekt der Biogasgewinnung ist die Nutzung zur Einspeisung in ein der Biogasanlage zugeordnetes Blockheizkraftwerk für eine wirtschaftlich lukrative Erzeugung von Elektroenergie, welche über staatlich festgelegte Vergütungssätze gefördert und unterstützt wird.

Die Erzeugung von Biogas erfolgt bei der anaeroben Nassvergärung im wesentlichen in vier Schritten, der Hydrolyse, der Versäuerungsphase (Acidogenese), der Essigsäurebildung (Acetogenese) und der Methanogenese, wofür jeweils unterschiedliche Bakterienstämme zuständig sind.

Bei einer einstufigen Biogasanlage laufen die vorstehenden Prozesse ohne räumliche Trennung gemeinsam und parallel ab. Ein solcher stabiler, eingefahrener und kontinuierlicher Prozessverlauf wird erst nach einer Einfahrphase erreicht.

Der vorstehende, einstufige und kontinuierliche Betrieb zur Nassfermentation ist bei Verwendung des Durchflussverfahrens mit einem relativ einfachen und kostengünstigen Anlagenaufbau zu realisieren und durch Automatisierungsmöglichkeiten mit angemessenem Aufwand durchzuführen.

Dabei ist es für einen störungsfreien Gärprozess und Anlagenbetrieb wesentlich im Durchflussverfahren ein möglichst gleiches Füllniveau im Fermenter und gegebenenfalls in einem nachgeordneten Nachfermenter aufrecht zu erhalten. Dazu wird die mittels der Fütterungseinrichtung dem Fermenter zugeführte Beschickungsmenge als etwa gleiche, entsprechend der Verweildauer vergorene Substratmenge dem Fermenter entnommen. Diese Entnahme von Entnahmesubstrat erfolgt in bekannter Weise durch einen Überlauf oder durch einen Pumpvorgang in einen Nachfermenter oder in ein Endlager. Ohne diese Entnahme wäre der Fermenter durch die ständige Fütterung in Kürze unzulässig überfüllt. Im Entnahmesubstrat ist das Substrat wegen der begrenzten Verweildauer im Fermenter nicht voll vergoren.

Es ist zudem allgemein bekannt, dass einem Fermenter ein Separator nachgeordnet werden kann, mit dem das für einen Niveauausgleich abgezogene Entnahmesubstrat zumindest teilweise in einen Substrat-Feststoffanteil und einen Substrat-Flüssigkeitsanteil separiert wird. Aus dem abgezogenen Entnahmesubstrat wird ein hoher Substrat-Feststoffanteil separiert und nicht mehr einem Endlager zugeführt. Damit werden im Endlager Schwimmschichten reduziert und/oder dort Rührvorgänge reduziert, sowie die Ausbringung eines flüssigeren Endlagerinhalts mit weniger Feststoffanteilen vereinfacht. Der separierte Substrat-Feststoffanteil wird somit hier aus dem Biogasprozess vollständig herausgenommen und anschließend kompostiert oder als Dünger ausgebracht.

Zudem ist es allgemein bekannt, dass die Biogasausbeute von mehreren Faktoren abhängig ist. Wesentliche Faktoren sind dabei insbesondere das Energie- und Vergärpotential der verfütterten Biomasse sowie das Verarbeitungspötential und die Arbeitsleistung der Bakterien. Dieses Verarbeitungspotential hängt dabei wesentlich davon ab, dass sich die Bakterien bei guter Gesundheit, hoher Lebensdauer und hoher Vermehrungsrate voll Appetit der angebotenen Fütterungsbiomasse zuwenden und diese in den vorstehend genannten vier Verarbeitungsschritten zu Biogas mit hohem Methangehalt verarbeiten. Besonders kritisch und sensibel sind dabei die Methanbakterien, welche für den letzten Prozessschritt, die Methanogenese zuständig sind. Es ist bereits bekannt das Umfeld der Bakterien in einem Fermenter zur Erhöhung deren Arbeitsleistung und damit für eine hohe Biogaserzeugung positiv zu beeinflussen, indem die Temperatur auf einem bestimmten Temperaturniveau gehalten wird und/oder der PH-Wert und/oder weitere Prozessparameter überwacht und geregelt werden.

Aufgabe der Erfindung ist es, ein Herstellverfahren zur Herstellung eines Biomasse-Futters für Bakterien sowie ein Biomasse-Futter für Bakterien vorzuschlagen, welches zur Steigerung der Gasausbeute in einer Biogasanlage verwendet werden kann. Eine weitere Aufgabe der Erfindung ist es, ein Betriebsverfahren für eine Biogasanlage unter Verwendung eines solchen Biomasse-Futters vorzuschlagen.

Die Aufgabe bezüglich des Herstellverfahrens zur Herstellung eines Biomasse-Futters für Bakterien wird mit den Merkmalen des Anspruchs 1 gelöst.

Gemäß Anspruch 1 wird in einem Fermenter einer einstufigen, im Durchflussverfahren arbeitenden Biogasanlage ein Substrat mit einem Trockensubstanzgehalt in einem Nassfermentationsprozess vergoren. Ein solches Substrat wird als Entnahmesubstrat dem Fermenter während des Betriebs insbesondere in Verbindung mit einer Fütterung zur Stabilisierung des Substratniveaus im Fermenter entnommen und zumindest teilweise einem Separator zugeführt, in dem eine zumindest teilweise Trennung in einen Substrat-Feststoffanteil und einen Substrat-Flüssiganteil erfolgt. In einem weiteren Verfahrensschritt wird zumindest einem Teil des separierten Substrat-Feststoffanteils wenigstens ein Futterergänzungsmittel in der Art eines Mittels zur Erhöhung des Nährstoff-/Energiepotentials und/oder eines Mittels zur Appetitanregung für die Bakterien und/oder eines Mittels zur Gesundheitsförderung für die Bakterien zugesetzt.

Es wird somit unter Verwendung des Substrat-Feststoffanteils ein hochwertiges Biomasse-Futter in der Art eines Bio-Kraftfutters für Bakterien hergestellt, das die mögliche Energieausbeute und/oder den Appetit und damit die Arbeitsbereitschaft der Bakterien und/oder deren Gesundheit und Wohlbefinden in Verbindung mit einer geeigneten Lebensdauer und Vermehrung positiv beeinflusst und damit die Effektivität und die Wirtschaftlichkeit einer Biogasanlage durch eine hohe Gasausbeute verbessert. Weiter wird dabei berücksichtigt, dass bei dem im Durchflussverfahren aus dem Fermenter zwangsläufig abgezogenen Entnahmesubstrat noch ein relativ hoher Anteil weiter vergärbarer Feststoffanteile enthalten sind. Dieser Feststoffanteil wird im Separator separiert und bildet mit den darin auch in erheblichem Umfang noch enthaltenen Bakterien die Basis für das erfindungsgemäße Bio-Kraftfutter für Bakterien. Somit enthält bereits dieses Basismaterial erhebliches Energiepotential und Vergärpotential, welches weiter durch die Zugabe und gezielte Zudosierung mit den vorstehend genannten Futterergänzungsmitteln weiter erhöht werden kann. Weiter werden durch Futterergänzungsmittel die Bakterien hinsichtlich ihres Wohlbefindens, ihrer Gesundheit und ihrer Fresslust unmittelbar positiv beeinflusst. Das mit Futterergänzungsmitteln angereicherte Bio-Kraftfutter kann insbesondere wegen der relativ großen Basismenge aus dem Substrat-Feststoffanteil einfach gehandhabt und gezielt wieder einer Biogasanlage, insbesondere mit der normalen Fütterungseinrichtung zugeführt werden.

Gemäß Anspruch 2 werden als bevorzugte Futterergänzungsmittel Mineralstoffe und/oder Enzyme und/oder Vitamine vorgeschlagen, welche von den Bakterien mit dem Futter aufgenommen werden und diese positiv beeinflussen. Gegebenenfalls können als Futterergänzungsmittel auch vorgezüchtete, substratspezifische Bakterien, welche den aktuellen Fütterungsstoffen angepasst sind, zudosiert werden.

Gemäß Anspruch 3 sollen Futterergänzungsmittel dem Substrat-Feststoffanteil in einem bestimmten Gewichts- oder Volumenverhältnis zugesetzt werden. Ein solches günstiges Verhältnis kann empirisch ermittelt und vorgegeben werden. Eine weitere Optimierung wird erreicht, wenn dieses Verhältnis je nach dem aktuellen Fütterungsstoff und/oder aktueller Zustandsparameter im Fermenter variiert wird. Die Zudosierung der Futterergänzungsmittel zum Substrat-Feststoffanteil kann zur Einhaltung der Gewichts- oder Volumenverhältnisse in an sich bekannter Weise automatisiert werden.

Gemäß Anspruch 4 eignen sich als Separatoren Schrauben- bzw. Schneckenseparatoren und/oder Zentrifugenseparatoren und/oder Siebbandpressen.

Eine besondere Variante eines Separators als Bio-Separator wird mit den Merkmalen des Anspruchs 5 beansprucht. Hier wird in einem Biofeststofffilter aus Biofeststoffen der Substrat-Feststoffanteil separiert und aufgenommen. Futterergänzungsmittel werden hier dann dem Material des Biofeststofffilters mit dem darin aufgenommenen Substrat-Feststoffanteil zudosiert, wodurch ein Bio-Kraftfutter erhalten wird.

Mit Anspruch 6 wird ein Biomasse-Futter für Bakterien als Bio-Kraftfutter beansprucht, welches nach einem der vorstehenden Herstellverfahren hergestellt ist. Ein solches Bio-Kraftfutter kann als Schüttgut oder in Gebinden für den Handel und Verkauf sowie die anschließende Verfütterung in Biogasanlagen zur Verfügung gestellt werden.

Alternativ dazu wird als Lösung hinsichtlich des Betriebsverfahrens für eine Biogasanlage das hergestellte Bio-Kraftfutter in einem Kreislauf wieder einem Fermenter der Anlage zugeführt, wobei eine Futterergänzungsvorrichtung mit einer Misch- und Transporteinrichtung sowie einer Zudosiereinrichtung für eine Zudosierung von Futterergänzungsmitteln Bestandteil der Biogasanlage sind. Mit der hier vorgeschlagenen Weiterbildung und gegebenenfalls Aufrüstung einer an sich bekannten Biogasanlage durch einen Separator und durch eine Futterergänzungsvorrichtung für die Zudosierung von Futterergänzungsmitteln kann die Effektivität der Biogasanlage und damit deren wirtschaftlicher Betrieb erhöht werden.

Der am Separator anfallende Substrat-Flüssiganteil kann für eine weitere Verwendung als Flüssigdünger einem Endlager zugeführt werden, wobei wegen des separierten und anderweitig verwendeten Substrat-Feststoffanteils dort ungünstige Schwimmschichten vermieden werden. Zudem kann der anfallende Substrat-Flüssiganteil bei Bedarf auch wieder dem Fermenter zugeführt werden.

Das Betriebsverfahren kann nach Anspruch 10 auch bei einer Biogasanlage mit mehreren Fermentern durchgeführt werden, wobei das dem Separator zugeführte Entnahmesubstrat einem Vorfermenter und/oder einem Nachfermenter entnommen werden kann. Das erzeugte Bio-Kraftfutter wird vorzugsweise wieder in einen Vorfermenter eingespeist.

Anhand einer Zeichnung wird die Erfindung näher erläutert.

Es zeigen:
- Fig. 1: eine schematische Draufsicht auf eine Biogasanlage und
- Fig. 2: eine Biogasanlage ähnlich Fig. 1 mit einem speziellen Separator, der schematisch in einer Seitenansicht dargestellt ist.

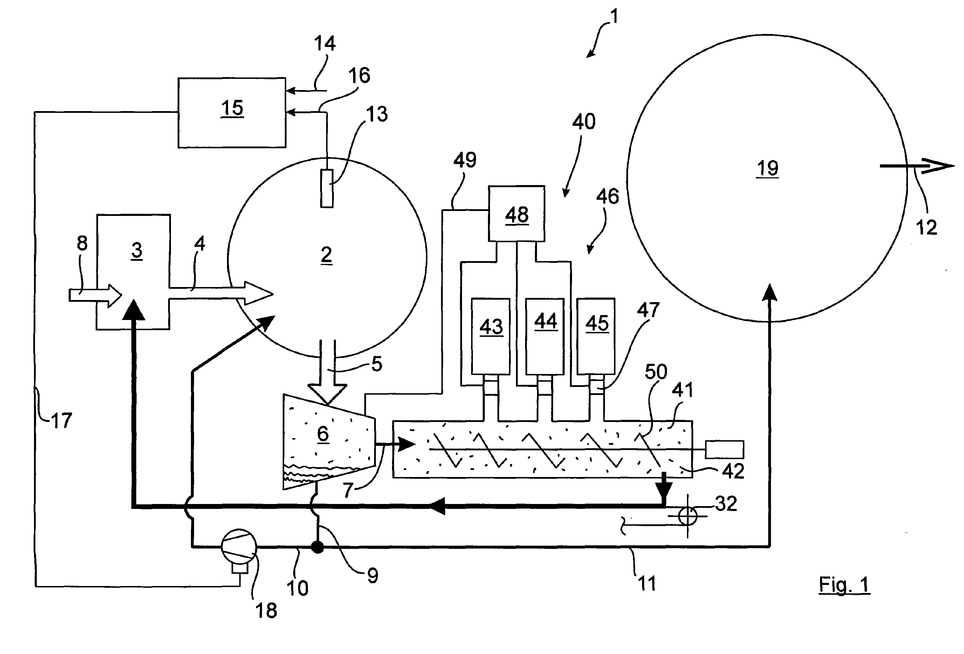

In Fig. 1 ist eine Biogasanlage 1 in einer schematischen Draufsicht gezeigt mit einem Fermenter 2 und einem Endlager 19. Dem Fermenter 2 ist eine Fütterungseinrichtung 3 vorgeschaltet, mit der kontinuierlich oder chargenweise vergärbare Stoffe dem Fermenter 2 über (nicht im Einzelnen dargestellte) Fördermittel 4 zugeführt werden.

Zur Stabilisierung des Flüssigkeitsniveaus im Fermenter 2 wird von dort eine der Fütterungsmenge entsprechende Menge an Substrat als Entnahmesubstrat durch Verdrängen oder Pumpen über eine (schematisch angedeutete) Förderleitung 5 entnommen und hier insgesamt einem Separator 6 beispielsweise einem Schraubenseparator oder einer Siebbandpresse zugeführt.

Das über die Förderleitung 5 abgezogene Entnahmesubstrat wird in einer Fest-Flüssig-Trennung im Separator 6 separiert zu einem Substrat-Feststoffanteil (schematisch im oberen Teil des Separators dargestellt) und einem Substrat-Flüssigkeitsanteil (schematisch im unteren Teil des Separators 6 dargestellt).

Der Substrat-Feststoffanteil wird vom Separator 6 ausgeworfen (schematisch durch Pfeil 7 dargestellt) und einer Futterergänzungsvorrichtung 40 für die Zugabe und Anreicherung sowie Vermischung mit Futterergänzungsmitteln zugeführt. Die Futterergänzungsvorrichtung umfasst hier eine rohrförmige, horizontal liegende Misch- und Transporteinrichtung 41 in der eine Misch- und Förderschnecke 50 drehangetrieben angeordnet ist. Der Substrat-Feststoffanteil wird entsprechend Pfeil 7 hier an einer (linken) Stirnseite der Misch- und Transporteinrichtung 41 zugeführt, gemischt und an einem gegenüberliegenden Auslass 42 auf ein Fördermittel, hier schematisch als Teil eines Förderbands 32 dargestellt, ausgeworfen.

Über der Misch- und Transporteinrichtung 41 und mit dieser verbunden sind Vorratsgefäße 43, 44, 45, die Teil einer Zudosiereinrichtung 46 sind. In den Vorratsgefäßen 43, 44, 45 sind jeweils Futterergänzungsmittel wie Mineralstoffe, Enzyme oder Vitamine in Pulver- oder Granulatform oder flüssig enthalten. Gegebenenfalls könnte in einem weiteren Vorratsgefäß zudem noch ein speziell vorgezüchtetes Bakteriensubstrat für eine Zudosierung vorgehalten werden.

Den Vorratsgefäßen 43, 44, 45 sind jeweils in den Verbindungsleitungen zur Misch- und Transporteinrichtung 41 Dosierelemente 47 nachgeordnet, beispielsweise steuerbare Schieber oder steuerbare Dosierpumpen. Diese Dosierelemente sind jeweils mit einer Steuer- und Regeleinheit 48 elektrisch verbunden, mit der entsprechend einem vorgegebenen Gewichts- oder Volumenverhältnis bezüglich des transportierten Substrat-Feststoffanteils eine Zudosierung der einzelnen Futterergänzungsmittel erfolgt, welche in der Misch- und Transporteinrichtung in das dabei gebildete Bio-Kraftfutter eingemischt und weitertransportiert werden. Als Basis für die jeweilige Menge eines zudosierten Futterergänzungsmittels wird hier der Steuer- und Regeleinheit 48 über eine Datenleitung 49 die vom Separator 6 separierte und der Misch- und Transporteinrichtung 41 zugeführte Menge an Substrat-Feststoffanteilen mitgeteilt. Zudem oder alternativ könnte die Menge des anzureichernden Substrat-Feststoffanteils durch andere an sich bekannte Maßnahmen ermittelt werden, wie durch eine Gewichtsbestimmung oder die aus der Drehung der Schnecke 50 abgeleitete Fördermenge.

Das nach der Anreicherung mit den Futterergänzungsmitteln hergestellte Bio-Kraftfutter wird über das Förderband 32 insgesamt der Fütterungseinrichtung 3 wieder zugeführt und dort zusammen mit zugegebenen vergärbaren Stoffen (Pfeil 8), insbesondere nachwachsenden Rohstoffen (NaWaRos) für eine erneute Beschickung in den Fermenter 2 bereitgehalten. Damit steht insgesamt ein hochwertiges Biomasse-Futter für die Bakterien zur Verfügung, wodurch die Effektivität der Biogaserzeugung gesteigert wird.

Der separierte Substrat-Flüssigkeitsanteil wird im oder nach dem Separator 6 aufgefangen und als Efluent über die Flüssigkeitsleitung 9 abgeführt. Diese verzweigt sich in eine Flüssigkeitsleitung 10, welche über ein Dosierpumpe 18 für eine teilweise Wiedereinspeisung des Substrat-Flüssigkeitsanteils zum Fermenter 2 führt und in eine Flüssigkeitsleitung 11, mit dem der nicht dem Fermenter 2 zugeführte Substrat-Flüssigkeitsanteil in das Endlager 19 gelangt. Von dort kann das gelagerte Efluent insbesondere als Flüssigdünger ausgebracht werden (Pfeil 12). Vorteilhaft sind in diesem Flüssigdünger wenig Feststoffanteile enthalten, so dass ein sonst ungünstiges Anhaften an Pflanzen mit einem Verkleben und Verschließen von Poren und Kapillaren weitgehend vermieden wird.

Zudem ist im Fermenter 2 eine schematisch dargestellte Sonde 13 zur Erfassung eines Ist-Trockensubstanzgehalts eingebracht, deren Messwert als IstWert (Pfeil 16) einem Regler 15 zugeführt ist, an dem zudem ein Soll-Trockensubstanzgehalt (Pfeil 14) vorgebbar ist. Bei einer Regelabweichung wird vom Regler 15 über eine Steuerleitung 17 ein bewertetes Stellsignal für eine ermittelte Mengenzudosierung eines Substrat-Flüssiganteils an die Dosierpumpe 18 abgegeben. Dadurch kann gegebenenfalls auf eine Zudosierung von Fremdflüssigkeit verzichtet werden. Zudem kann hierüber auch auf die Fütterung mit Biofeststoffen aus der Fütterungseinrichtung 3 regelnd eingegriffen werden.

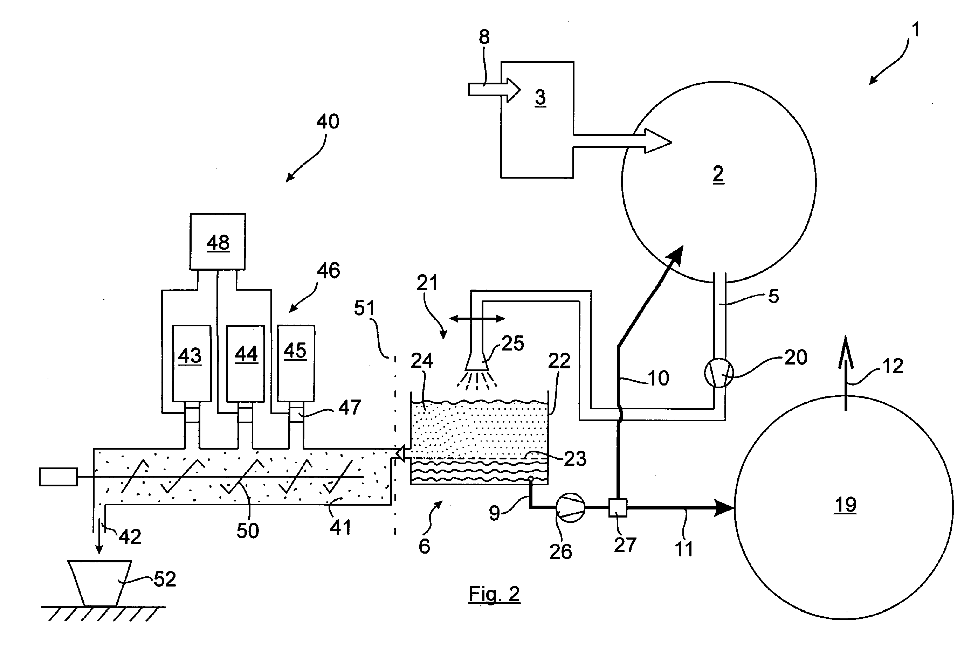

Figur 2 zeigte eine ähnliche Biogasanlage 1, jedoch ohne Feststoff-Regeleinrichtung. In dieser Biogasanlage 1 ist ein spezieller Separator 6 als Bio-Separator verwendet, der in einer schematischen Seitenansicht dargestellt ist. Für gleiche Teile, die Figur 1 entsprechen, sind gleiche Bezugszeichen verwendet.

Auch in der Biogasanlage 1 nach Fig. 2 sind ein Fermenter 2, eine Fütterungseinrichtung 3 und ein Endlager 19 vorgesehen. Vom Fermenter 2 wird wie in der Ausführung nach Fig. 1 bei einer Fütterung zum Niveauausgleich im Fermenter 2 über die Förderleitung 5 eine entsprechende Menge an Entnahmesubstrat abgeführt und hier mit einer Pumpe 20 dem Separator 6 zugeführt, der als Bio-Separator 21 bezeichnet wird. Beim Bio-Separator 21 ist in einen wannenartigen Separatorbehälter 22 auf einem Gittenzwischenboden 23 eine Schüttung 24 aus Biofeststoffen, beispielsweise eine Strohschüttung, eingebracht. Über diese wird durch einen gegebenenfalls in der Fläche bewegbaren Ausströmer 25 das Entnahmesubstrat geleitet. Dadurch wird in der Schüttung 24 der Substrat-Feststoffanteil aus dem Entnahmesubstrat weitgehend zurückgehalten und separiert. Unterhalb des Gitterzwischenbodens 22 sammelt sich der durchsickernde separierte Substrat-Flüssigkeitsanteil und wird über die Flüssigkeitsleitung 9 durch die Pumpe 26 weggepumpt. Nach der Pumpe 26 ist in der Leitung ein Dreiwegeventil 27 eingesetzt, so dass je nach dessen Stellung ein bestimmter Substrat-Flüssigkeitsanteil über die Flüssigkeitsleitung 10 dem Fermenter 2 oder über die Flüssigkeitsleitung 11 dem Endlager 19 zugeführt werden kann.

Aus dem Bio-Separator wird zur Herstellung eines Bio-Kraftfutters die Schüttung 24 zusammen mit dem dort zurückgehaltenen Substrat-Feststoffanteil ausgebracht und einer Futterergänzungsvorrichtung 40 zugeführt, wie sie im Aufbau und der Funktion im Zusammenhang mit Fig. 1 bereits beschrieben wurde.

Die Ausbringung der Schüttung 24 mit dem zurückgehaltenen Substrat-Feststoffanteil aus dem Separatorbehälter 22 kann jeweils chargenweise oder bei einer kontinuierlichen Nachsteuerung der Schüttung 24 auch kontinuierlich erfolgen. Dazu könnte beispielsweise der Gitterzwischenboden 23 in einer weiteren Funktion als angetriebener Kratzboden ausgeführt sein mit einer Schiebebewegung in Richtung auf eine Auslassöffnung.

In Fig. 2 ist dem Bio-Separator 21 die Futterergänzungsvorrichtung 40 für eine direkte Beschickung unmittelbar nachgeordnet. Die Futterergänzungsvorrichtung 40 könnte jedoch auch an einem anderen Ort stehen und nicht Bestandteil der Biogasanlage 1 sein, was durch die Trennlinie 51 schematisch angedeutet ist. Für diesen Fall müsste die Schüttung 24 mit dem zurückgehaltenen Substrat-Feststoffanteil zur Futterergänzungsvorrichtung 40 transportiert werden. Zur Futterergänzungsvorrichtung 40 könnten dann von mehreren Biogasanlagen separierte Substrat-Feststoffanteile gegebenenfalls mit Schüttung 24 zur Herstellung von Bio-Kraftfutter geliefert werden.

Am Ausgang 42 tritt das hergestellte Kraftfutter nach der Zudosierung der Futterergänzungsmittel aus und wird in Gebinden 52 oder als Schüttgut für eine Weiterverwendung zur Fütterung einer Biogasanlage zur Verfügung gestellt.

## Patentansprüche

1. Herstellverfahren zur Herstellung eines Biomasse-Futters für Bakterien, bei dem
- in einem Fermenter (2) einer einstufigen, im Durchflussverfahren arbeitenden Biogasanlage (1) ein Substrat mit einem Trockensubstanzgehalt in einem Nassfermentationsprozess vergoren wird, wobei
- Substrat als Entnahmesubstrat dem Fermenter (2) entnommen wird,
- das Entnahmesubstrat zumindest teilweise einem Separator (6) zugeführt wird, in dem eine zumindest teilweise Trennung in einen Substrat-Feststoffanteil und einen Substrat-Flüssiganteil erfolgt, und
- in einem weiteren Verfahrensschritt zumindest einem Teil des separtierten Substrat-Feststoffanteils wenigstens ein Futterergänzungsmittel in der Art eines Mittels zur Erhöhung des Nährstoff-/ Energiepotentials und/oder eines Mittels zur Appetitanregung für die Bakterien und/oder eines Mittels zur Gesundheitsförderung für die Bakterien zugesetzt wird, wodurch ein hochwertiges Biomasse-Futter als Bio-Kraftfutter für Bakterien gebildet wird.

2. Herstellverfahren nach Anspruch 1, **dadurch gekennzeichnet, dass** dem Substrat-Feststoffanteil als Futterergänzungsmittel Mineralstoffe und/oder Enzyme und/oder Vitamine zugemischt werden, sowie gegebenenfalls eine Zudosierung mit vorgezüchteten, substratspezifischen Bakterien erfolgt.

3. Herstellverfahren nach Anspruch 1 oder 2, **dadurch gekennzeichnet, dass** Futterergänzungsmittel dem Substrat-Feststoffanteil in einem bestimmten Gewichts- oder Volumenverhältnis zugesetzt werden, wobei das Gewichts- oder Volumenverhältnis fest vorgegeben oder in Abhängigkeit des vorgesehenen Fütterungseinsatzes, insbesondere von Zustandsparametern in einem Fermenter (2) vorgegeben wird.

4. Herstellverfahren nach einem der Ansprüche 1 bis 3, **dadurch gekennzeichnet, dass** als Separator (6) ein Schrauben- bzw. Schneckenseparator und/oder ein Zentrifugenseparator und/oder eine Siebbandpresse verwendet wird.

5. Herstellverfahren nach einem der Ansprüche 1 bis 3, **dadurch gekennzeichnet, dass** der Separator (6) als Bio-Separator (21) aufgebaut ist und eine Schüttung (24) aus Biofeststoffen als Biofeststofffilter enthält, über das das Entnahmesubstrat geleitet wird, wodurch der Substrat-Feststoffanteil im Biofeststofffilter separiert und aufgenommen wird und unter dem Feststofffilter der separierte Substrat-Flüssiganteile für die weitere Verarbeitung aufgefangen wird, und
dass anschließend dem Material des Biostofffilters mit dem darin aufgenommenen Substrat-Feststoffanteil Futterergänzungsmittel zum Erhalt eines Bio-Kraftfutters zugesetzt werden.

6. Biomasse-Futter für Bakterien als Bio-Kraftfutter nach einem der Ansprüche 1 bis 5.

7. Biomasse-Futter nach Anspruch 6, **dadurch gekennzeichnet, dass** das Biomasse-Futter als Bio-Kraftfutter für Bakterien als Schüttgut oder in Gebinden für den Handel und die Verfütterung in Biogasanlagen zur Verfügung gestellt wird.

8. Betriebsverfahren für eine Biogasanlage unter Verwendung eines Biomasse-Futters nach den Ansprüchen 6 oder 7, welches nach einem der Herstellverfahren der Ansprüche 1 bis 5 hergestellt ist,
- bei dem in einem Fermenter (2) ein Substrat mit einem Trockensubstanzgehalt in einem Nassfermentationsprozess vergoren wird, wobei mit einer Fütterungseinrichtung (3) eine vorgegebene Menge an vergärbaren Stoffen kontinuierlich oder chargenweise in den Fermenter (2) eingebracht wird und eine entsprechende Menge an Entnahmesubstrat dem Fermenter (2) durch Verdrängung oder Pumpen entnommen wird,
- in einem Separator (6) zumindest eine Teilmenge des Entnahmesubstrats in einen Substrat-Feststoffanteil und einen Substrat-Flüssiganteil separiert wird, wobei der Separator (6) als Bestandteil der Biogasanlage (1) dem Fermenter (2) nachgeordnet ist,
- der Substrat-Feststoffanteil nach dem Separator (6) kontinuierlich oder chargenweise einer Futterergänzungsvorrichtung (40) zugeführt wird, die eine Misch- und Transporteinrichtung (41) aufweist, der eine Zudosiereinrichtung (46) für eine Zudosierung von Futterergänzungsmitteln, wie Mineralstoffe und/oder Enzyme und/oder Vitamine und/oder substratspezifische Bakterien zugeordnet ist, wobei die Misch- und Transporteinrichtung (41) und die Zudosiereinrichtung (46) Bestandteile der Biogasanlage (1) sind, und
- das am Ausgang (42) der Misch- und Transporteinrichtung verfügbare Biomasse-Futter als Bio-Kraftfutter direkt gegebenenfalls über eine Vorgrube als Vorratsgefäß und/oder über die Fütterungseinrichtung (3) wieder in den Fermenter (2) eingespeist wird.

9. Betriebsverfahren nach Anspruch 8, **dadurch gekennzeichnet, dass** der am Separator (6) anfallenden Substrat-Flüssiganteil zumindest teilweise wieder dem Fermenter (2) oder zumindest teilweise einem Endlager (19) zugeführt wird.

10. Betriebsverfahren nach Anspruch 8 oder 9, **dadurch gekennzeichnet, dass** die Biogasanlage wenigstens zwei Fermenter und ein Endlager (19) aufweist und das dem Separator (6) zugeführte Entnahmesubstrat einem Vorfermenter und/oder einem Nachfermenter entnommen wird und dass das Bio-Kraftfutter in einen Vorfermenter eingespeist wird.
